(19) Europäisches Patentamt
European Patent Office
Office européen des brevets

(11) **EP 2 614 817 A1**

(12) **EUROPEAN PATENT APPLICATION**

(43) Date of publication:
**17.07.2013 Bulletin 2013/29**

(51) Int Cl.:
**A61K 9/16** (2006.01)      **A61K 9/20** (2006.01)
**A61K 9/50** (2006.01)

(21) Application number: **13000090.4**

(22) Date of filing: **09.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.01.2012 JP 2012003626**

(71) Applicant: **NITTO DENKO CORPORATION Osaka 567 (JP)**

(72) Inventors:
• **Shishido, Takuya**
  **Ibaraki-shi, Osaka, 567-8680 (JP)**
• **Asari, Daisuke**
  **Ibaraki-shi, Osaka, 567-8680 (JP)**
• **Hori, Mitsuhiko**
  **Ibaraki-shi, Osaka, 567-8680 (JP)**

(74) Representative: **Müller-Boré & Partner**
  **Patentanwälte**
  **Grafinger Straße 2**
  **81671 München (DE)**

(54) **Pharmaceutical allergen composition and method for producing the same**

(57) The present invention provides a pharmaceutical composition capable of preserving and delivering an allergen having poor thermal stability without using gelatin as a stabilizer, and a method for producing the pharmaceutical composition.

The present invention provides a pharmaceutical composition containing an allergen and additives selected from at least two groups from among group (A) consisting of polysaccharides having high formability; group (B) consisting of mono- to hexasaccharides, sugar alcohols thereof, maltodextrin, and polyvinylpyrrolidone; and group (C) consisting of viscous polysaccharides; wherein the additives have a stabilizing effect on the allergen.

EP 2 614 817 A1

**Description**

The present invention relates to a pharmaceutical composition useful as an agent for preventing or treating allergy symptoms. More specifically, the present invention relates to a pharmaceutical composition having excellent stability of an allergen and excellent usability in terms of storage, handling, and the like, and a method for producing the same.

Current treatments for allergic diseases such as a pollen allergy are mostly symptomatic treatments with antihistamines, but recent attention has been focused on hyposensitization therapy as a possible curative treatment of allergic diseases.

Most of the current preparations for specific hyposensitization therapy are subcutaneous injections, and these preparations usually must be administered for a long period of time (about 2 to 3 years). In this regard, a dosage form that can improve the quality of life (QOL) of caregivers and patients is believed to be necessary.

Specific hyposensitization therapy with subcutaneous injections has problems such as a risk of anaphylactic shock, the need for administration by a healthcare professional, the need for long-term, frequent hospital visits, pain associated with injection, and the need for refrigeration storage.

In contrast, liquid and tablet preparations for sublingual administration have been recently marketed in Europe and the United States and receiving attention because of their convenience and reduced side effects.

However, specific hyposensitization therapy by sublingual administration of liquid preparations still has problems such as inaccuracy of the dose and the need for refrigeration storage.

Specific hyposensitization therapy by sublingual administration of tablets has problems such as accidental ingestion, difficulty in controlling the dose, poor portability, and unpleasant sensation in the oral cavity due to residue.

In the development of allergen preparations, it is essential that allergens are stably preserved, i.e., that the loss of the biological activity is minimized.

As a technique to develop such an allergen preparation, a method that uses a lyophilized preparation containing a stabilizer and an excipient has been suggested.

For example, Patent Literature 1 suggests a pharmaceutical composition containing stabilized timothy grass pollen allergen, obtained by lyophilizing a solution containing gelatin and mannitol or starch and mannitol as stabilizers. Additionally, for example, Patent Literature 2 suggests a pharmaceutical composition containing a stabilized recombinant protein of a major cedar pollen allergen, obtained by lyophilizing a solution containing mannitol as a stabilizer and acetic acid as a pH adjuster. Additionally, Patent Literature 3 suggests a pharmaceutical composition containing a stabilized recombinant protein of a major mite allergen, obtained by lyophilizing a solution containing macrogol 4000, polysorbate-80, and sucrose.

However, with these conventional techniques to develop allergen preparations, it has been still difficult to stably preserve and deliver allergens due to their poor thermal stability.

Patent Literature 1: JP-T 2006-513269
Patent Literature 2: JP-B 3932272
Patent Literature 3: JP-A 2007-277094

In view of the existing problems described above, the present inventors first developed a pharmaceutical composition containing a stabilized major cedar pollen allergen, by lyophilizing a solution containing gelatin and an organic acid salt as stabilizers.

However, improvements were needed in the following points: gelatin may induce an allergic reaction in immunized people, possibly inducing serious side effects such as anaphylactic shock; and gelatin may be unusable depending on a raw material thereof due to a religious belief that prohibits eating products from animals such as pigs and cows.

In view of the situation described above, the present invention aims to provide a pharmaceutical composition capable of stably preserving and delivering allergens having poor thermal stability without using gelatin as a stabilizer; and a method for producing the pharmaceutical composition.

In order to solve the above problems, the present inventors conducted intensive studies, and as a result, found that even allergens having poor thermal stability can be stably preserved and delivered by a composition containing a specific sugar, a polysaccharide, an edible polymer, and the like as additives. The present invention was accomplished based on such a finding.

Specifically, the present invention relates to a pharmaceutical composition containing an allergen and additives selected from at least two groups from among group (A) consisting of polysaccharides having high formability; group (B) consisting of mono- to hexasaccharides, sugar alcohols thereof, maltodextrin, and polyvinylpyrrolidone; and group (C) consisting of viscous polysaccharides; wherein the additives have a stabilizing effect on the allergen.

The pharmaceutical composition of the present invention is preferably free of water.

The additive selected from the polysaccharides having high formability in group (A) is preferably at least one selected from the group consisting of pectin, dextran, starch, and pullulan.

The additive selected from the mono- to hexasaccharides and sugar alcohols thereof in group (B) is preferably at least one selected from the group consisting of glucose, mannose, raffinose, trehalose, maltitol, isomalt, and sorbitol.

The additive selected from the viscous polysaccharides in group (C) is preferably at least one selected from the group consisting of guar gum, tara gum, locust bean gum, xanthan gum, tamarind gum, $\tau$-carrageenan, and gellan gum.

The allergen is preferably a cedar pollen allergen protein.

The pharmaceutical composition of the present invention preferably further contains an organic acid salt.

Another aspect of the present invention is a method for producing a pharmaceutical composition, the method including preparing an allergen-containing preparation solution in which an allergen and additives are dissolved in water, and lyophilizing the allergen-containing preparation solution; wherein the additives have a stabilizing effect on the allergen and are selected from at least two groups from group (A) consisting of polysaccharides having high formability; group (B) consisting of mono- to hexasaccharides, sugar alcohols thereof, maltodextrin, and polyvinylpyrrolidone; and group (C) consisting of viscous polysaccharides.

In the method for producing the pharmaceutical composition of the present invention, the allergen-containing preparation solution preferably has a pH in the range of 5.0 to 9.0.

The present invention is described in detail below.

The pharmaceutical composition of the present invention contains an allergen and additives.

The additives are formed from non-gelatin materials and are edible polymers. They are ingredients as the base materials of the pharmaceutical composition of the present invention.

In the pharmaceutical composition of the present invention, the additives are selected from at least two groups from among group (A) consisting of polysaccharides having high formability; group (B) consisting of mono- to hexasaccharides, sugar alcohols thereof, maltodextrin, and polyvinylpyrrolidone; and group (C) consisting of viscous polysaccharides.

Use of the additives selected from at least two groups from among group (A), group (B), and group (C), i.e., combined use of multiple additives selected from at least two groups described above enables long-term and stable preservation and delivery of an allergen. The reason is assumed as follows: while additives having different physical properties such as molecular weight and solubility in water result in different three-dimensional structures formed after lyophilization and different interactions with allergens, the present invention uses multiple additives selected from two or more groups described above, whereby each additive enhances the stabilizing effect on an allergen in a synergistic manner without interfering with each other.

In the pharmaceutical composition of the present invention, the additives have a stabilizing effect on the allergen. In regard to the expression "stabilizing effect on the allergen" as used herein, the additives are considered to have a stabilizing effect on an allergen when a medicament-containing composition prepared by dissolving an allergen and additives in water and removing the water by lyophilization exhibits an allergenic activity of 75% or more after being stored at 40 $\pm$ 2°C for 7 days. Examples of additives having the stabilizing effect on an allergen include those having high glass-transition temperatures. Additives having low glass-transition temperatures are crystallized during lyophilization, which is believed to result in the inactivation of the protein of the allergen.

The "polysaccharides having high formability in group (A)" refer to polysaccharides with which a dosage form having usable properties can be obtained when an aqueous solution containing such a polysaccharide as an additive is lyophilized.

Any polysaccharide can be used without limitation. For example, at least one selected from the group consisting of pectin, dextran, starch, and pullulan is suitably used. These polysaccharides are substances soluble in water, and preferably have a molecular weight of 10,000 or more.

The term "molecular weight" as used herein refers to a weight average molecular weight, and is a value that can be obtained by gel permeation chromatography analysis.

The pectin has a molecular weight of about 30,000 to 100,000. It is usually a polymeric polysaccharide extracted with water from citrus fruits or apples, and is composed of galacturonic acid and the methyl ester thereof.

The pectin is classified into LM pectin and HM pectin, according to the degree of methyl esterification. In the present invention, either pectin may be used. However, because LM pectin forms a thermally irreversible gel in the presence of calcium ion, use of LM pectin in combination with an organic acid salt containing calcium ions is not preferred.

The amount of pectin is preferably 0.1 to 20% by weight, more preferably 0.5 to 10% by weight, based on the total weight of an allergen-containing preparation solution (described later) immediately before lyophilization in the production process. With less than 0.1% by weight, an adequate dosage form as a medicinal product may not be formed after lyophilization. In contrast, with more than 20% by weight, the viscosity of the allergen-containing preparation solution will be very high, which may cause problems in the production.

Usually, the dextran is a partially decomposed polysaccharide produced by sucrose fermentation with *Leuconostoc mesenteroides Van Tieghem* (*Lactobacillaceae*), and is mainly composed of D-glucose.

The dextran can be used without any particular problems as long as the average molecular weight is 10, 000 or more.

From the viewpoint that the present invention is for medical use, dextran 40 (average molecular weight of 40,000) or dextran 70 (average molecular weight of 70,000) listed in Japanese Pharmaceutical Excipients is suitably used.

The amount of dextran is preferably 0.1 to 30% by weight, more preferably 0.5 to 20% by weight, based on the total weight of the allergen-containing preparation solution immediately before lyophilization in the production process. With less than 0.1% by weight, an adequate dosage form as a medicinal product may not be formed after lyophilization. In contrast, with more than 30% by weight, the dextran may not be uniformly dispersed or dissolved in the allergen-containing preparation solution, which may cause problems in the production.

In the pharmaceutical composition of the present invention, in the case where a polysaccharide having high formability selected from group (A) is pullulan, the amount thereof is preferably 0.1 to 50% by weight, more preferably 0.5 to 40% by weight, based on the total weight of the allergen-containing preparation solution immediately before lyophilization in the production process. With less than 0.1% by weight, an adequate dosage form as a medicinal product may not be formed after lyophilization. In contrast, with more than 50% by weight, the viscosity of the allergen-containing preparation solution will be very high, which may cause problems in the production.

In the case where a polysaccharide having high formability selected from group (A) is starch, the amount thereof is preferably 0.1 to 50% by weight, more preferably 0.5 to 40% by weight, based on the total weight of the allergen-containing preparation solution immediately before lyophilization in the production process. With less than 0.1% by weight, an adequate dosage form as a medicinal product may not be formed after lyophilization. In contrast, with more than 50% by weight, the viscosity of the allergen-containing preparation solution will be very high, which may cause problems in the production.

Group (B) consists of mono- to hexasaccharides, sugar alcohols thereof, maltodextrin, and polyvinylpyrrolidone (hereinafter also referred to as PVP).

An additive selected from the mono- to hexasaccharides and sugar alcohols thereof is preferably at least one selected from the group consisting of glucose, mannose, raffinose, trehalose, maltitol, isomalt, and sorbitol.

Usually, the maltodextrin is partially hydrolyzed corn or potato starch, and is a mixture of monomers, dimers, oligomers, and polymers of glucose. The percentage of each ingredient constituting the maltodextrin varies depending on the degree of hydrolysis. Accordingly, herein, partially hydrolyzed starch having a dextrose equivalent of 1 to 20 is defined as maltodextrin.

The polyvinylpyrrolidone (PVP) is a synthetic polymer mainly consisting of linear 1-vinyl-2-pyrrolidone groups. The PVP is a polymer whose molecular weight varies depending on the degree of polymerization, and its property is determined based on the relative viscosity of PVP to water in the aqueous solution, which is represented by the K value of 10 to 120.

From the viewpoint that the present invention is for medical use, PVP K25, PVP K30, and PVP K90 listed in Japanese Pharmaceutical Excipients are suitably used as the PVP.

In the pharmaceutical composition of the present invention, the amount of the additive of group (B) is preferably 0.1 to 80% by weight, more preferably 1.0 to 80% by weight, based on the total weight of the allergen-containing preparation solution immediately before lyophilization in the production process. With less than 0.1% by weight, sufficient stability of the allergen may not be achieved after lyophilization. In contrast, with more than 80% by weight, the viscosity of the allergen-containing preparation solution will be very high, which may cause problems in the production.

The viscous polysaccharides of group (C) show viscosity when dissolved in water and are not included in group (A). In other words, when an additive of group (C) is used alone, a dosage form with no problems in use cannot be obtained after lyophilization of the allergen-containing preparation solution.

Any type of viscous polysaccharides may be used without limitation. For example, polysaccharides containing glucose and mannose are preferred. Examples of such polysaccharides preferably include at least one selected from the group consisting of galactomannans such as guar gum, tara gum, and locust bean gum, xanthan gum, tamarind gum, $\tau$-carrageenan, and gellan gum.

The gellan gum is a natural, linear heteropolysaccharide produced in an extracellular form from *Sphingomonas elodea;* is composed of a repeating tetrasaccharide unit of glucose, glucuronic acid, glucose, and rhamnose; and is classified into native gellan gum and deacylated gellan gum depending on the presence of an acetyl group and a glyceryl group on 1,3-linked glucose residues.

The native gellan gum has a gelation temperature of about 80°C, and is not suitable in the case where the allergen-containing preparation solution contains an allergen having low thermal stability. On the other hand, deacylated gellan gum is preferably used in the present invention because deacylated gellan gum does not undergo gelation unless a cation is added.

The gellan gum forms a thermally irreversible gel in the presence of cations, particularly, calcium ion. Thus, use of the gellan gum in combination with organic acid salts including calcium ion is not preferred.

The amount of the additive of group (C) is preferably 0.1 to 3.0% by weight, more preferably 0.5 to 2.0% by weight, based on the total weight of the allergen-containing preparation solution immediately before lyophilization in the production process. With less than 0.1% by weight, an adequate dosage form as a medicinal product may not be formed after lyophilization. In contrast, with more than 3.0% by weight, the viscosity of the allergen-containing preparation solution will be very high, or the additive may not be uniformly dispersed, thus causing problems in the production.

The pharmaceutical composition of the present invention may also contain an appropriate amount of an edible polymer soluble only in water or an edible polymer insoluble in both water and organic solvents (hereinafter these edible polymers are also collectively referred to as "other edible polymers") in combination with the above-described additives, as long as the effects of the present invention are not impaired.

The amount of the other edible polymers is preferably 0.1 to 10% by weight based on the total weight of the pharmaceutical composition of the present invention.

The allergen refers to an antigen to which an antibody of a person with allergic disease specifically reacts. The allergy is typically a protein.

Specific examples include allergens from pollen of trees (golden acacia, red alder, white ash, American beech, birch, box elder, mountain cedar, red cedar, common cottonwood, cypress, American elm, Chinese elm, Japanese Douglas fir, sweet gum, eucalyptus, hackberry, hickory, linden, sugar maple, mesquite, mulberry, oak, olive, pecan tree, pepper tree, pine, common privet, Russian olive, American sycamore, tree of heaven, black walnut, black willow, etc.); allergens from pollen of grasses (cotton, Bermuda grass, Kentucky bluegrass, smooth brome, cultivated corn, meadow fescue, Johnson grass, cultivated oats, orchard grass, redtop, perennial rye grass, rice, sweet vernal grass, timothy, careless weed, pigweed, common cocklebur, sorrel dock, goldenrod, kochia, lamb's quarters, marigold, nettle, pigwood, English plantain, giant ragweed, short ragweed, western ragweed, Russian thistle, sagebrush, Scotch broom, sheep sorrel, etc.); allergens from insects (silkworms, mites, honeybees, wasps, ants, cockroaches, etc.); allergens from fungi (*Alternaria tenuis, Aspergillus fumigatus, Botrytis cinerea, Candida albicans, Cephalosporium acremonium, Curvularia spicifera, Epicoccum nigrum, Epidermophyton floccosum, Fusarium vasinfectum, Helminthosporium interseminatum, Hormodendrum cladosporioides, Mucor rasemosus, Penicillium notatum, Phoma herbarium, Pullularia pullulans, Rhizopus nigricans,* etc.); allergens from fur of animals (dogs, cats, birds, etc.); allergens from house dust; and allergens from foods. The allergen is not particularly limited as long as it is an allergen with which an antibody of a person with allergic disease specifically reacts.

Today, there is a demand for hyposensitization therapy for cedar pollen allergy that afflicts many people.

Therefore, in the pharmaceutical composition of the present invention, the allergen is preferably cedar pollen allergen protein.

Examples of the cedar pollen allergen protein include those containing, as an active ingredient, at least one selected from the group consisting of antigenic proteins extracted from cedar pollen with which an antibody of a person with allergic disease specifically reacts, and proteins having high homology with the above proteins at the amino acid level.

Examples of the protein having antigenicity from the cedar pollen include proteins that are contained in cedar pollen and capable of inducing the production of cedar pollen-specific IgE antibodies. These proteins contained in cedar pollen include major cedar pollen allergen proteins and minor cedar pollen allergen proteins.

In several cedar pollen extracts included in pollen, components to which a majority of patients are highly sensitized are referred to as the major cedar pollen allergen proteins, while components to which only some of the patients are sensitized are referred to as the minor cedar pollen allergen proteins.

The cedar pollen allergen proteins may be in a liquid or solid form containing such proteins. The cedar pollen allergen protein in a liquid form is referred to as a cedar pollen extract. When a liquid cedar pollen extract is used, the pharmaceutical composition of the present invention can be used as-is as an injectable solution or an oral solution. The pharmaceutical composition of the present invention may also be transformed into a gel as an oral solid preparation. Particularly preferred as the cedar pollen extracts are Cryj1 and Cryj2, which are the major cedar pollen allergen proteins, and a mixture thereof. A cedar pollen extract containing not only Cryj1 and Cryj2 but also minor cedar pollen allergen proteins is also preferably used as-is or in a dilute or lyophilized solid form.

Although the amount of the allergen varies depending on its properties and the like, usually, a preferred amount is $1 \times 10^{-10}$ to 60% by weight based on the total amount of the pharmaceutical composition of the present invention. With less than $1 \times 10^{-10}$% by weight, the resulting product may not be suitable for hyposensitization therapy. With more than 60% by weight, the strength of a preparation containing the pharmaceutical composition of the present invention may be significantly reduced, causing problems in terms of shape retainability.

Further, the pharmaceutical composition of the present invention is preferably such that the aqueous solution of the allergen-containing preparation immediately before lyophilization in the production process has a pH of 5.0 to 9.0. With a pH in this range, it is possible to prevent a significant reduction in physicochemical stability of the allergen and thereby ensure the safety. A more preferred pH range is 6.0 to 8.0.

The pharmaceutical composition of the present invention preferably includes a pH adjuster in order to adjust the pH of the aqueous solution of the allergen-containing preparation in the above range.

The pH adjuster is not particularly limited. Examples thereof include those proven to be useful as pharmaceutical additives, such as adipic acid, aqueous ammonia, hydrochloric acid, sodium carbonate, dilute hydrochloric acid, citric acid hydrate, glycine, glucono-δ-lactone, gluconic acid, sodium dihydrogen phosphate (crystal), succinic acid, acetic acid, ammonium acetate, sodium acetate hydrate, diisopropanolamine, tartaric acid, potassium hydroxide, calcium hydroxide, sodium hydroxide, magnesium hydroxide, sodium hydrogen carbonate, sodium carbonate hydrate, triisopropanolamine, triethanolamine, carbon dioxide, lactic acid, sodium lactate solution, glacial acetic acid, fumaric acid, monosodium fumarate, sodium propionate, boric acid, ammonium borate, borax, maleic acid, anhydrous citric acid, anhydrous sodium monohydrogen phosphate, anhydrous sodium dihydrogen phosphate, meglumine, methanesulfonic acid, monoethanolamine, sulfuric acid, aluminum potassium sulfate hydrate, DL-malic acid, phosphoric acid, trisodium phosphate, dipotassium phosphate, potassium dihydrogen phosphate, and sodium dihydrogen phosphate. Any of these pH adjusters may be used alone or in combination of two or more thereof.

The pH adjuster may be a combination of an organic acid and a salt, which form an organic acid salt equivalent to those listed above as examples of the pH adjuster used in the pharmaceutical composition. For examples, it may be a combination of an organic acid and a salt such as sodium chloride, calcium chloride, magnesium chloride, or potassium chloride.

From the viewpoint of production, the pH adjuster is preferably one capable of adjusting the pH with a small amount. Examples of such pH adjusters include hydrochloric acid and sodium hydroxide.

Organic acids and organic acid salts effective in inhibiting denaturation and aggregation of proteins are also preferred as the pH adjuster. Examples of such pH adjusters include citric acid hydrate, glycine, glucono-$\delta$-lactone, gluconic acid, succinic acid, acetic acid, sodium acetate hydrate, tartaric acid, lactic acid, glacial acetic acid, fumaric acid, monosodium fumarate, sodium propionate, maleic acid, anhydrous citric acid, and malic acid.

The pharmaceutical composition of the present invention may further contain at least one organic acid salt that functions to improve the stability of the allergen.

Examples of such organic acid salts include amino acid salts, adipate, citrate, malate, acetate, succinate, propionate, butyrate, malonate, glutarate, maleate, glycolate, lactate, gluconate, fumarate, tartrate, glycyrrhizinate, and pimelate. These organic acid salts may be used alone or in any combination of two or more thereof.

Particularly preferred among the above organic acid salts are those proven to be useful as pharmaceutical additives and whose pH does not deviate from an optimal pH range (5.0 to 9.0) of the allergen-containing pharmaceutical composition when dissolved in water. Specific examples include calcium lactate, dipotassium glycyrrhizate, disodium glycyrrhizinate, trisodium glycyrrhizinate, sodium citrate, calcium gluconate, sodium gluconate, magnesium gluconate, disodium succinate, DL-sodium tartrate, L-sodium tartrate, and potassium sodium tartrate. These may be used alone or in combination of two or more.

Particularly preferred are calcium lactate, dipotassium glycyrrhizate, and sodium citrate from the viewpoint of the stabilizing effect on the allergen.

In the pharmaceutical.composition of the present invention, the amount of the organic acid salt is preferably 0.01 to 20% by weight, more preferably 0.1 to 10% by weight, based on the total weight of the allergen-containing preparation solution immediately before lyophilization in the production process. With less than 0.01% by weight, the resulting product may exhibit hardly any stabilizing effect on the allergen. In contrast, with more than 20% by weight, it may be difficult to control the physical properties of the pharmaceutical composition due to the added additives. Additionally, many organic acid salts have a peculiar taste, and it may cause problems in use, considering the fact that a preparation containing the pharmaceutical composition of the present invention is for oral administration.

The pharmaceutical composition of the present invention may further include an additive that improves the physical properties of the pharmaceutical composition and the stability and solubility of the allergen, for example, at least one selected from the group consisting of sugars, sugar alcohols, and sugar fatty acids, which do not belong to group (B) described above.

Examples of such sugars include monosaccharides, disaccharides, and tri- to hexasaccharides listed below.

Examples of monosaccharides include: aldotetroses such as erythrose and threose; aldopentoses such as ribose, lyxose, xylose, and arabinose; aldohexoses such as allose, talose, gulose, altrose, galactose, and idose; ketotetroses such as erythrulose; ketopentoses such as xylulose and ribulose; and ketohexoses such as psicose, fructose, sorbose, and tagatose. Examples of disaccharides include: $\alpha$-diglucosides such as kojibiose, nigerose, maltose, and isomaltose; $\beta$-diglucosides such as isotrehalose, sophorose, laminaribiose, cellobiose, and gentiobiose; $\alpha,\beta$-diglucocides such as neotrehalose; and isomaltulose (palatinose). Examples of tri- to hexasaccharides include cyclic oligosaccharides such as fructooligosaccharides, galactooligosaccharides, xylooligosaccharides, isomaltooligosaccharides, chitin oligosaccharides, chitosan oligosaccharides, oligoglucosamine, and cyclodextrins.

Examples of monosaccharide alcohols include: tetritols such as erythritol, D-threitol, and L-threitol; pentitols such as D-arabinitol and xylitol; hexitols such as D-iditol, galactitol (dulcitol), and mannitol; and cyclitols such as inositol. Examples of disaccharide alcohols include lactitol; and examples of oligosaccharide alcohols include pentaerythritol and reduced malt sugar starch syrup.

A sugar or sugar alcohol substitute may be used in the pharmaceutical composition of the present invention. The sugars and sugar alcohols can be used alone or in combination of two or more thereof.

The sugars or sugar alcohols are preferably mono- to trisaccharides or sugar alcohols thereof from the viewpoint of facilitating the dissolution of the pharmaceutical composition of the present invention in the oral cavity and preventing a significant change in the viscosity of the solution in the production process.

Examples of the sugar fatty acids include sorbitan fatty acid esters and sucrose fatty acid esters.

Examples of the sorbitan fatty acid esters include sorbitan monooleate, sorbitan trioleate, sorbitan sesquioleate, sorbitan cocoate, and polyoxyethylene sorbitan fatty acid esters.

Examples of the sucrose fatty acid esters include sucrose stearate, sucrose oleate, sucrose palmitate, sucrose myristate, sucrose behenate, sucrose erucate, and sucrose-mixed fatty acid esters.

These sugar fatty acids are very convenient because they are useful not only as stabilizers of proteins and peptides but also as defoamers.

In the pharmaceutical composition of the present invention, the amount of the above additives is preferably 1 to 80% by weight, more preferably 5 to 70% by weight, based on the total weight of the pharmaceutical composition of the present invention. With less than 1% by weight, sufficient physical properties for use may not be attained. In contrast, with more than 80% by weight, it may be difficult to control the physical properties of the pharmaceutical composition due to the added additives.

The pharmaceutical composition of the present invention may suitably contain, in addition to the above-described materials, the following additives as desired, as ingredients constituting the base materials: perfumes, flavoring substances, sweetening agents, colorants, antiseptics, antioxidants, other stabilizers, surfactants, and the like. These additives are not particularly limited and any known additive can be used.

The pharmaceutical composition of the present invention is preferably free of water.

Because the pharmaceutical composition of the present invention is free of water, there is no need, for example, for antiseptics or sterilization treatment required for general jelly preparations containing pharmaceutical compositions, and this gives advantage in terms of production costs. The pharmaceutical composition of the present invention is also suitably used in nutritional supplements for patients who require fluid restriction.

The expression "free of water" as used herein includes a case where the pharmaceutical composition is substantially free of water. For example, it means that the water content is 5% by weight or less, preferably 2.5% by weight or less, and more preferably 1% by weight or less, based on the total weight of the pharmaceutical composition of the present invention.

An oral solid preparation can be prepared using the pharmaceutical composition of the present invention. In addition to the above-mentioned materials, any of the following additives may be suitably used as desired: excipients, binders, perfumes, flavoring substances, sweetening agents, colorants, antiseptics, antioxidants, stabilizers, surfactants, and the like. These additives are not particularly limited and any known additive can be used.

Examples of the oral solid preparation include tablets, coated tablets, powders, granules, fine granules, orally disintegrating tablets, oral patches, jellies, and films. The oral solid preparation is not particularly limited as long as it is in a solid form for oral, sublingual, and buccal administration.

Such a pharmaceutical composition of the present invention is suitably used for oral hyposensitization therapy in which the sensitization time must be controlled; and is particularly suitable for sublingual hyposensitization therapy. Because the pharmaceutical composition of the present invention contains specific additives as stabilizers, it can stably maintain allergens, particularly proteins and peptides.

Further, the pharmaceutical composition of the present invention is a porous solid called cake, and is a lyophilized preparation produced by a method in which water used as a solvent in an aqueous solution containing an allergen is sublimated by lyophilization (described later).

The lyophilized preparation as the pharmaceutical composition of the present invention is physically stable from room temperature to about 60°C.

Additionally, because the pharmaceutical composition of the present invention mainly contains the above-described polysaccharide additives, it can be easily dissolved at a temperature close to the body temperature in the oral cavity in an amount of water in the oral cavity, and the physical properties for use can be significantly improved.

The pharmaceutical composition can also stably maintain an allergen protein, particularly cedar pollen allergen protein.

The pharmaceutical composition of the present invention may be directly swallowed, or may be immediately dissolved in the oral cavity and then swallowed. It is also possible to control the dissolution time in the oral cavity to allow absorption through oral or sublingual mucosa.

The pharmaceutical composition of the present invention significantly improves the QOL of patients and caregivers from the following viewpoints: it completely dissolves at about body temperature, thus leaving no sense of residue; and it is physically stable, thus making it easy for patients and caregivers to pick it up with the fingers.

Although the physical strength of the pharmaceutical composition of the present invention is not particularly limited, for example, the pharmaceutical composition preferably has a physical strength to resist physical damage such as cracks and chips when the preparation is packed, stored, transported, or handled by patients. Further, even when the preparation is held with the hands, such contact at about body temperature causes no melting of the preparation or deterioration in properties.

While a preparation containing the pharmaceutical composition of the present invention is physically stable, it also must be quickly broken down in the presence of water, for example, in contact with saliva in the mouth. The preparation is preferably broken down in the oral cavity within 150 seconds, more preferably within 60 seconds.

Although the size of the pharmaceutical composition of the present invention is not particularly limited, the pharmaceutical composition preferably has a planer area of 0.5 to 6. 0 cm$^2$. With a planar area of less than 0.5 cm$^2$, a preparation containing the pharmaceutical composition of the present invention may be difficult to handle when it is picked up with the fingers for administration. With a planer area of more than 6.0 cm$^2$, such a preparation may not be completely placed in the oral cavity, particularly under the tongue.

The pharmaceutical composition of the present invention can be prepared, for example, by a method including preparing an allergen-containing preparation solution in which an allergen and additives are dissolved in water, and lyophilizing the allergen-containing preparation solution.

Such a method for producing the pharmaceutical composition of the present invention is also one aspect of the present invention.

The additives described in the method for producing the pharmaceutical composition of the present invention are the same as those in the above-described pharmaceutical composition of the present invention.

The allergen-containing preparation solution prepared in the step of preparing an allergen-containing preparation solution preferably has a pH in the range of 5.0 to 9.0. With a pH of the allergen-containing preparation solution in this range, it is possible to prevent a significant reduction in the physicochemical stability of the allergen and thereby ensure the safety. In this step, other ingredients such as pH adjusters and additives may be added if necessary.

In the step of lyophilizing the allergen-containing preparation solution, for example, a predetermined amount of the allergen-containing aqueous solution is preferably dispensed at a temperature of 28 to 35°C into a blister of a desired size for lyophilization, and lyophilized immediately after being dispensed.

The resulting pharmaceutical composition is preferably formed into a finished product by being sealed in a package if necessary.

The pharmaceutical composition of the present invention prepared by the above method is a lyophilized preparation as described above, and is suitable as an oral solid preparation. The pharmaceutical composition after lyophilization has an excellent external appearance and good solubility in water for injection, and can maintain the stability of the allergen for a long period of time. Accordingly, it can also be used as an injectable solution or preparation for transmucosal (transnasal, oral, sublingual) administration.

Because the pharmaceutical composition of the present invention contains specific additives in addition to an allergen, it has excellent storage stability for preservation and delivery of the allergen.

Additionally, according to the method for producing the pharmaceutical composition of the present invention, even an allergen known to have very poor thermal stability can be stably maintained during production, and the resulting pharmaceutical composition also has excellent storage stability.

The present invention is specifically described with reference to the following examples, but is not limited to these examples.

Test Example Using Additive of Group (A) (polysaccharides having high formability)

(Test Example 1)

LM pectin (10 parts by weight, GENU PECTIN Type LM-102AS-J manufactured by CP Kelco) was added and dissolved, by heating if necessary, in purified water (989. 9 parts by weight). After the mixture was cooled to room temperature, 0.1 parts by weight of cedar pollen extract lyophilized powder (manufactured by LSL) was added to the mixture, sufficiently mixed, and dissolved at room temperature. The obtained preparation solution was dispensed in 1.0 g aliquots into a vial for lyophilization, and lyophilized to prepare a medicament-containing composition.

For a storage stability test, the obtained medicament-containing composition was stored at 40 $\pm$ 2°C for 30 days, and the allergenic activity at day 7, day 14, and day 30 of storage was evaluated by the method described below. Table 2 shows the results.

(Test Examples 2 to 6)

Preparation solutions were prepared with the compositions shown in Table 1 by the same procedure as in Test Example 1, and lyophilized to prepare medicament-containing compositions. In Test Example 2, HM pectin (GENU PECTIN Type USP-H manufactured by CP Kelco) was used. In Test Example 3, dextran 40 (manufactured by Wako Pure Chemical Industries, Ltd.) was used. In Test Example 4, dextran 70 (manufactured by Wako Pure Chemical Industries, Ltd.) was used. In Test Example 5, starch (manufactured by Wako Pure Chemical Industries, Ltd.) was used. In Test Example 6, pullulan (manufactured by Hayashibara Co., Ltd.) was used. The medicament-containing compositions obtained in Test Examples 2 to 6 were subjected to a storage stability test in the same manner as in Test Example 1. Table 2 shows the results.

(Allergenic Activity Evaluation Method)

The allergenic activity of Cry j 1, which is one of the major cedar pollen allergens, was measured using a cedar pollen antigen ELISA Kit "Cry j 1" (manufactured by Seikagaku Biobusiness Corporation). The principle of the measurement kit is a sandwich ELISA that uses monoclonal antibodies (013 and 053) specific to Cry j 1, which is one of Japanese Cedar *(Cryptomeria japonica)* pollen antigens. This kit can specifically measure Cry j 1.

A standard solution or a sample (20 μL) was added to a reaction buffer solution (100 μL) included in the kit, and a primary reaction was carried out at room temperature for 60 minutes. Then, an HRP-labeled antibody solution (100 μL) was added to the reaction product, and a secondary reaction was carried out for 60 minutes. An enzyme substrate solution (100 μL) was added thereto, and a reaction was carried out at room temperature and shielded from light for 30 minutes. Finally, a reaction stop solution (100 μL) was added thereto. Subsequently, the ultraviolet absorption intensity at 450 nm was measured. A calibration curve was determined based on the absorption intensity of the standard solution at various Cry j 1 concentrations, and the Cry j 1 allergenic activity (ng/mL) of each sample was determined based on the calibration curve.

The Cry j 1 allergenic activity % was determined after sampling the pharmaceutical compositions subjected to the storage stability test (at day 7, day 14, and day 30 of storage) and immediately after production (30 minutes and 60 minutes after production). The Cry j 1 allergenic activity % was evaluated based on the following scoring criteria.

5: 90% or more to less than 105%

4: 75% or more to less than 90%

3: 60% or more to less than 75%

2: 45% or more to less than 60%

1: 30% or more to less than 45%

0: less than 30%

Table 1

| Ingredients | Composition [parts by weight] | | | | | |
|---|---|---|---|---|---|---|
| | Test Examples | | | | | |
| | 1 | 2 | 3 | 4 | 5 | 6 |
| Lyophilized dry powder of cedar pollen extract | 0.1 | 0.1 | 0.1 | 0.1 | 0.1 | 0.1 |
| LM pectin | 10 | - | - | - | - | - |
| HM pectin | - | 10 | - | - | - | - |
| Dextran 40 | - | - | 10 | - | - | - |
| Dextran 70 | - | - | - | 10 | - | - |
| Starch | - | - | - | - | 10 | - |
| Pullulan | - | - | - | - | - | 10 |
| [Purified water] | [989.9] | [989.9] | [989.9] | [989.9] | [989.9] | [989.9] |
| Aliquot [g/vial] | 1.0 | 1.0 | 1.0. | 1.0 | 1.0 | 1.0 |
| Composition | Group A | | | | | |

Table 2

| Sample | | Remaining allergenic activity | | |
|---|---|---|---|---|
| | | Day 7 | Day 14 | Day 30 |
| Test Example 1 | Group A | 5 | 5 | 4 |
| Test Example 2 | | 5 | 4 | 4 |
| Test Example 3 | | 4 | 4 | 3 |
| Test Example 4 | | 4 | 4 | 3 |
| Test Example 5 | | 4 | 4 | 3 |
| Test Example 6 | | 4 | 3 | 3 |

Test Example Using Additive of Group (B) (consisting of sugars, sugar alcohols, maltodextrin, and PVP)

(Test Example 7)

Glucose (10 parts by weight, manufactured by Wako Pure Chemical Industries, Ltd.) was added and dissolved, by heating if necessary, in purified water (989.9 parts by weight). After the mixture was cooled to room temperature, cedar pollen extract lyophilized powder (0.1 parts by weight, manufactured by LSL) was added to the mixture, sufficiently mixed, and dissolved at room temperature. The obtained preparation solution was dispensed in 1.0 g aliquots into a vial for lyophilization, and lyophilized to prepare a medicament-containing composition. For a storage stability test, the obtained medicament-containing composition was stored at 40 ± 2°C for 30 days, and the allergenic activity at day 7, day 14, and day 30 of storage was evaluated by the same method as in Test Example 1. Table 4 shows the results.

(Test Examples 8 to 17)

Preparation solutions were prepared with the compositions shown in Table 3 by the same procedure as in Test Example 7, and lyophilized to prepare medicament-containing compositions. In Test Example 8, mannose (manufactured by Wako Pure Chemical Industries, Ltd.) was used. In Test Example 9, trehalose (manufactured by Hayashibara Co., Ltd.) was used. In Test Example 10, raffinose (manufactured by Wako Pure Chemical Industries, Ltd.) was used. In Test Example 11, maltitol (manufactured by Hayashibara Biochemical Laboratories, Inc.) was used. In Test Example 12, isomalt (Galen 800 manufactured by BENEO-Palatinit GmbH) was used. In Test Example 13, sorbitol (manufactured by Roquette) was used. In Test Example 14, maltodextrin (AMICOL 10 manufactured by Nippon Starch Chemical Co., Ltd.) was used. In Test Example 15, PVP K25 (manufactured by Wako Pure Chemical Industries, Ltd.) was used. In Test Example 16, PVP K30 (manufactured by Wako Pure Chemical Industries, Ltd.) was used. In Test Example 17, PVP K90 (Wako Pure Chemical Industries, Ltd.) was used. The medicament-containing compositions obtained in Test Examples 8 to 17 were subjected to a storage stability test in the same manner as in Test Example 1. Table 4 shows the results.

(Comparative Test Example 1)

Cedar pollen extract lyophilized powder (0.1 parts by weight, manufactured by LSL) was added to purified water (999.9 parts by weight), and dissolved at room temperature. Subsequently, the resultant mixture was quickly dispensed in 1.0 g aliquots into a vial for lyophilization, and lyophilized to prepare a medicament-containing composition. The obtained medicament-containing composition was stored at 40 ± 2 °C, and the allergenic activity at day 7, day 14, and day 30 of storage was evaluated by the same method as in Test Example 1. Table 4 shows the results.

Test Examples Using Group (B') (additives that are sugars but have no stabilizing effect on cedar pollen allergens)

(Comparative Test Examples 2 to 6)

Preparation solutions were prepared with the compositions shown in Table 3 by the same procedure as in Test Example 7, and lyophilized to prepare medicament-containing compositions. In Comparative Test Example 2, mannitol (manufactured by Roquette) was used. In Comparative Test Example 3, erythritol (Wako Pure Chemical Industries, Ltd.) was used. In Comparative Test Example 4, xylitol (manufactured by Wako Pure Chemical Industries, Ltd.) was used.

In Comparative Test Example 5, polyethyleneglycol 4000 (PEG4000 manufactured by Wako Pure Chemical Industries, Ltd.) was used. In Comparative Test Example 6, polyethyleneglycol 20000 (PEG20000 manufactured by Wako Pure Chemical Industries, Ltd.) was used. PEG was used as an example to be tested in comparison to PVP, which is also a water-soluble polymer. The medicament-containing compositions obtained in Comparative Test Examples 2 to 6 were subjected to a storage stability test in the same manner as in Test Example 1. Table 4 shows the results.

Table 3

| Ingredients | Composition [parts by weight] | | | | | | | | | | | | | | | | |
| | Test Examples | | | | | | | | | | | Comparative Test Examples | | | | | |
| | 7 | 8 | 9 | 10 | 11 | 12 | 13 | 14 | 15 | 16 | 17 | 1 | 2 | 3 | 4 | 5 | 6 |
| Lyophilized dry powder of cedar pollen extract | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Glucose | 10 | - | - | - | - | - | - | - | - | - | - | - | - | | - | - | - |
| Mannose | - | 10 | - | - | - | - | - | - | - | - | - | - | - | - | - | - | - |
| Trehalose | - | - | 10 | - | - | - | - | - | - | - | - | - | - | - | - | - | - |
| Raffinose | - | - | - | 10 | - | - | - | - | - | - | - | - | - | - | - | - | - |
| Maltitol | - | - | - | - | 10 | - | - | - | - | - | - | - | - | - | - | - | - |
| Isomalt | - | - | - | - | - | 10 | - | - | - | - | - | - | - | - | - | - | - |
| Sorbitol | - | - | - | - | - | - | 10 | - | - | - | - | - | - | - | - | - | - |
| Maltodextrin | - | - | - | - | - | - | - | 10 | - | - | - | - | - | - | - | - | - |
| PVP K25 | - | - | - | - | - | - | - | - | 10 | - | - | - | - | - | - | - | - |
| PVP K30 | - | - | - | - | - | - | - | - | - | 10 | - | - | - | - | - | - | - |
| PVP K90 | - | - | - | - | - | - | - | - | - | - | 10 | - | - | - | - | - | - |
| Mannitol | - | - | - | - | - | - | - | - | - | - | - | - | 10 | - | - | - | - |
| Erythritol | - | - | - | | - | - | - | - | - | - | - | - | - | 10 | - | - | - |
| Xylitol | - | - | - | - | - | - | - | - | - | - | - | - | - | - | 10 | - | - |
| PEG4000 | - | - | - | - | - | - | - | - | - | - | - | - | - | - | - | 10 | - |
| PEG20000 | - | - | - | - | - | - | - | - | - | - | - | -- | - | - | - | - | 10 |
| [Purified water] | [989.9] | [989.9] | [989.9] | [989.9] | [989.9] | [989.9] | [989.9] | [989.9] | [989.9] | [989.9] | [989.9] | [999.9] | [989.9] | [989.9] | [989.9] | [989.9] | [989.9] |
| Aliquot [g/vial] | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |

(continued)

| Ingredients | Composition [parts by weight] | | | | | | | | | | | | | | | | |
|---|---|---|---|---|---|---|---|---|---|---|---|---|---|---|---|---|---|
| | Test Examples | | | | | | | | | | | Comparative Test Examples | | | | | |
| | 7 | 8 | 9 | 10 | 11 | 12 | 13 | 14 | 15 | 16 | 17 | 1 | 2 | 3 | 4 | 5 | 6 |
| Composition | Group B | | | | | | | | | | | - | Group [B] | | | | |

Table 4

| Sample | | Remaining allergenic activity | | |
|---|---|---|---|---|
| | | Day 7 | Day 14 | Day 30 |
| Test Example 7 | Group B | 5 | 4 | 4 |
| Test Example 8 | | 5 | 4 | 3 |
| Test Example 9 | | 5 | 4 | 4 |
| Test Example 10 | | 5 | 5 | 4 |
| Test Example 11 | | 5 | 4 | 4 |
| Test Example 12 | | 5 | 4 | 3 |
| Test Example 13 | | 4 | 4 | 3 |
| Test Example 14 | | 4 | 4 | 4 |
| Test Example 15 | | 5 | 4 | 4 |
| Test Example 16 | | 5 | 4 | 4 |
| Test Example 17 | | 5 | 4 | 4 |
| Comparative Test Example 1 | - | 2 | 1 | 0 |
| Comparative Test Example 2 | Group [B'] | 2 | 1 | 0 |
| Comparative Test Example 3 | | 2 | 1 | 0 |
| Comparative Test Example 4 | | 0 | 0 | 0 |
| Comparative Test Example 5 | | 3 | 1 | 0 |
| Comparative Test Example 6 | | 3 | 0 | 0 |

As shown in Table 4, the sugars and sugar alcohols shown in Test Examples 7 to 17 were found to act as allergen stabilizers during lyophilization. On the other hand, the results show that the medicament-containing compositions of Comparative Test Examples in which mannitol and the like reportedly capable of stabilizing other allergens and vaccines were used were not necessarily effective against cedar pollen allergens. In regard to the water-soluble synthetic polymers, PVP was found to show a high stabilizing effect on the allergen.

Test Example Using Additive of Group (C) (viscous polysaccharides)

(Test Example 18)

Guar gum (10 parts by weight, MEYRO-GUAR CSA200/50, manufactured by DANISCO) was added and dissolved, by heating if necessary, in purified water (989. 9 parts by weight). After the mixture was cooled to room temperature, cedar pollen extract lyophilized powder (0.1 parts by weight, manufactured by LSL) was added to the mixture, sufficiently mixed, and dissolved at room temperature. The obtained preparation solution was dispensed in 1.0 g aliquots into a vial for lyophilization, and lyophilized to prepare a medicament-containing composition.
For a storage stability test, the medicament-containing composition was stored at 40 ± 2°C for 30 days, and the allergenic activity at day 7, day 14, and day 30 of storage was evaluated by the same method as in Test Example 1. Table 6 shows the results.

(Test Examples 19 to 24)

Preparation solutions were prepared with the compositions shown in Table 5 by the same procedure as in Test Example 18, and lyophilized to prepare medicament-containing compositions. In Test Example 19, locust bean gum (GENUGUM RL-200-J manufactured by CP Kelco) was used. In Test Example 20, xanthan gum (Echo-gum T manufactured by DSP Gokyo Food & Chemical Co., Ltd.) was used. In Test Example 21, tamarind gum (Glyloid 3S manufactured by DSP GOKYO FOOD & CHEMICAL CO., LTD.) was used. In Test Example 22, tara gum (MT120 manufactured by MRC Polysaccharide Co., Ltd.) was used. In Test Example 23, τ-carrageenan (CP Gum FA manufactured by DSP GOKYO FOOD & CHEMICAL CO., LTD.) was used. In Test Example 24, deacylated gellan gum (Kelcogel manufactured by CP Kelco) was used. The medicament-containing compositions obtained in Test Examples 19 to 24 were subjected to a storage stability test in the same manner as in Test Example 1. Table 6 shows the results.

 Test Examples Using Group (C') (additives that are viscous polysaccharides but have no stabilizing effect on the cedar pollen allergen)

(Comparative Test Examples 7 and 8)

Preparation solutions were prepared with the compositions shown in Table 5 by the same procedure as in Test Example 18, and lyophilized to prepare medicament-containing compositions. In Comparative Test Example 7, sodium alginate (Kimica Algin IL-6 manufactured by KIMICA corporation) was used. In Comparative Test Example 8, κ-carrageenan (GENUGEL JPE-126 manufactured by CP Kelco) was used. The medicament-containing compositions obtained in Comparative Test Examples 7 and 8 were subjected to a storage stability test in the same manner as in Test Example 1. Table 6 shows the results.

Table 5

| Ingredients | Composition [parts by weight] | | | | | | | | |
|---|---|---|---|---|---|---|---|---|---|
| | Test Examples | | | | | | | Comparative Test Examples | |
| | 18 | 19 | 20 | 21 | 22 | 23 | 24 | 7 | 8 |
| Lyophilized dry powder of cedar pollen extract | 0.1 | 0.1 | 0.1 | 0.1 | 0.1 | 0.1 | 0.1 | 0.1 | 0.1 |
| Guar gum | 10 | - | - | - | - | - | - | - | - |
| Locust bean gum | - | 10 | - | - | - | - | - | - | - |
| Xanthan gum | - | - | 10 | - | - | - | - | - | - |
| Tamarind gum | - | - | - | 10 | - | - | - | - | - |
| Tara gum | - | - | - | - | 10 | - | - | - | - |
| τ-Carrageenan | - | - | - | - | - | 10 | - | - | - |
| Deacylated gellan gum | - | - | - | - | - | - | 10 | - | - |
| Alginate Na | - | - | - | - | - | - | - | 10 | - |
| κ-Carrageenan | - | - | - | - | - | - | - | - | 10 |
| [Purified water] | [989.9] | [989.9] | [989.9] | [989.9] | [989.9] | [989.9] | [989.9] | [989.9] | [989.9] |
| Aliquot [g/vial] | 1.0 | 1.0 | 1.0 | 1.0 | 1.0 | 1.0 | 1.0 | 1.0 | 1.0 |
| Composition | Group C | | | | | | | Group [C'] | |

Table 6

| Sample | | Remaining allergenic activity | | |
|---|---|---|---|---|
| | | Day 7 | Day 14 | Day 30 |
| Test Example 18 | Group C | 5 | 5 | 4 |
| Test Example 19 | | 5 | 5 | 4 |
| Test Example 20 | | 5 | 4 | 4 |
| Test Example 21 | | 5 | 4 | 4 |
| Test Example 22 | | 4 | 4 | 4 |
| Test Example 23 | | 4 | 4 | 3 |
| Test Example 24 | | 4 | 3 | 3 |
| Comparative Test Example 7 | Group [C'] | 3 | 2 | 1 |
| Comparative Test Example 8 | | 3 | 0 | 0 |

As shown in Table 6, the gelling agents used in Test Examples 18 to 24 were found to act as allergen stabilizers during lyophilization. Among these gelling agents, guar gum, locust bean gum, xanthan gum, tamarind gum, and tara gum were found to show a high stabilizing effect on the allergen.

```
Group (A) + Group (B) (polysaccharides having formability +
sugars, maltodextrin, PVP, etc.)
```

(Example 1)

LM pectin (30 parts by weight) and glucose (10 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.

Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2°C for 90 days, and the allergenic activity after storage was evaluated by the same method as in Test Example 1. The pharmaceutical composition was also evaluated for its properties and solubility in water by the methods described below. Table 9 shows the results expressed as scores.

(Evaluation of Properties)

The obtained pharmaceutical composition was evaluated based on the following criteria. Thereafter, it was stored at 40 ± 2°C for 3 months, and evaluated again after storage. Table 9 shows the results.
3: There are no problems in use
1: There are problems in use

(Evaluation of Solubility in Water)

Purified water (10.0 g) heated to 37°C was added to the obtained pharmaceutical composition (1.0 g), and the dissolution of the pharmaceutical composition was observed at room temperature and evaluated based on the following criteria. Table 9 shows the results.
3: Completely dissolved within 60 seconds.

2: Completely dissolved in about 1 to 5 minutes.
1: It took 5 minutes or more until complete dissolution.

(Examples 2 to 10)

Allergen-containing preparation solutions were prepared with the compositions shown in Table 7 by the same procedure as in Example 1, and lyophilized to prepare pharmaceutical compositions. The pharmaceutical compositions obtained in Examples 2 to 10 were evaluated in the same manner as in Example 1, and the results were expressed as scores. Table 9 shows the results.

(Example 11)

Dextran 40 (60 parts by weight) and raffinose (10 parts by weight) were added to purified water (800 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.
Subsequently, the obtained preparation solution was quickly dispensed in 1.0 g aliquots into a vial for lyophilization, and lyophilized to prepare a pharmaceutical composition. The obtained pharmaceutical composition was evaluated in the same manner as in Example 1, and the results were expressed as scores. Table 9 shows the results.

(Examples 12 to 18)

Solutions were prepared with the compositions shown in Table 7 by the same procedure as in Example 11, and lyophilized to prepare pharmaceutical compositions. The pharmaceutical compositions obtained in Examples 12 to 18 were evaluated in the same manner as in Example 1, and the results were expressed as scores. Table 9 shows the results.

Table 7

| Ingredients | Composition [parts by weight] | | | | | | | | | | | | | | | | | |
|---|---|---|---|---|---|---|---|---|---|---|---|---|---|---|---|---|---|---|
| | Examples | | | | | | | | | | | | | | | | | |
| | 1 | 2 | 3 | 4 | 5 | 6 | 7 | 8 | 9 | 10 | 11 | 12 | 13 | 14 | 15 | 16 | 17 | 18 |
| Lyophilized dry powder of cedar pollen extract | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| LM pectin | 30 | 30 | 30 | 30 | 30 | 30 | 30 | 30 | - | - | - | - | - | - | - | - | - | - |
| HM pectin | - | - | - | - | - | - | - | - | 30 | 30 | - | - | - | - | - | - | - | - |
| Dextran 40 | - | - | - | - | - | - | - | - | - | - | 60 | 60 | - | - | - | - | - | - |
| Dextran 70 | - | - | - | - | - | - | - | - | - | - | - | - | 60 | 60 | - | - | - | - |
| Starch | - | - | - | - | - | - | - | - | - | - | - | - | - | - | 60 | 60 | - | - |
| Pullulan | - | - | - | - | - | - | - | - | - | - | - | - | - | - | - | - | 90 | 90 |
| Glucose | 10 | - | - | - | - | - | - | - | - | - | - | - | - | - | - | - | - | - |
| Raffinose | - | 10 | - | - | - | - | - | - | 10 | - | 10 | - | 10 | - | 10 | - | 10 | - |
| Trehalose | - | - | 10 | - | - | - | - | - | - | - | - | - | - | - | - | - | - | - |
| Makitol | - | - | - | 10 | - | - | - | - | - | - | - | - | - | - | - | - | - | - |
| Isomalt | - | - | - | - | 10 | - | - | - | - | - | - | - | - | - | - | - | - | - |
| Sorbitol | - | - | - | - | - | 10 | - | - | - | - | - | - | - | - | - | - | - | - |
| Maltodextrin | - | - | - | - | - | - | 10 | - | - | - | - | - | - | - | - | - | - | - |
| PVP K25 | - | - | - | - | - | - | - | 10 | - | 10 | - | 10 | - | 10 | - | 10 | - | 10 |
| Purified water | [959.9] | [959.9] | [959.9] | [959.9] | [959.9] | [959.9] | [959.9] | [959.9] | [959.9J | [959.9] | [929.9] | [929.9] | [929.9] | [929.9] | [929.9] | [929.9] | [889.9] | [889.9] |
| Aliquot [g/vial] | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |
| Composition | Groups A + B | | | | | | | | | | | | | | | | | |

**EP 2 614 817 A1**

At Least One Additive from Groups (A) and (B), or Group (A) + Group (B') (polysaccharides having formability + additives that are sugars but have no stabilizing effect)

(Comparative Example 1)

LM pectin (30 parts by weight) was added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.
Subsequently, the obtained preparation solution was quickly dispensed in 1.0 g aliquots into a vial for lyophilization, and lyophilized to prepare a pharmaceutical composition. The obtained pharmaceutical composition was evaluated in the same manner as in Example 1, and the results were expressed as scores. Table 9 shows the results.

(Comparative Examples 2 to 10)

Solutions were prepared with the compositions shown in Table 8 by the same procedure as in Comparative Example 1, and lyophilized to prepare pharmaceutical compositions. The pharmaceutical compositions obtained in Comparative Examples 2 to 10 were evaluated in the same manner as in Example 1, and the results were expressed as scores. Table 9 shows the results.

(Comparative Example 11)

LM pectin (30 parts by weight) and mannitol (10 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.
Subsequently, the obtained preparation solution was quickly dispensed in 1.0 g aliquots into a vial for lyophilization, and lyophilized to prepare a pharmaceutical composition. The obtained pharmaceutical composition was evaluated in the same manner as in Example 1, and the results were expressed as scores. Table 9 shows the results.

(Comparative Examples 12 to 14)

Solutions were prepared with the compositions shown in Table 8 by the same procedure as in Comparative Example 11, and lyophilized to prepare a pharmaceutical composition. The pharmaceutical compositions obtained in Comparative Examples 12 to 14 were evaluated in the same manner as in Example 1, and the results were expressed as scores. Table 9 shows the results.

Table 8

| Ingredients | Composition [parts by weight] | | | | | | | | | | | | | |
|---|---|---|---|---|---|---|---|---|---|---|---|---|---|---|
| | Comparative Examples | | | | | | | | | | | | | |
| | 1 | 2 | 3 | 4 | 5 | 6 | 7 | 8 | 9 | 10 | 11 | 12 | 13 | 14 |
| Lyophilized dry powder of cedar pollen extract | 0.1 | 0.1 | 0.1 | 0.1 | 0.1 | 0.1 | 0.1 | 0.1 | 0.1 | 0.1 | 0.1 | 0.1 | 0.1 | 0.1 |
| LM pectin | 30 | - | - | - | - | - | - | - | - | - | 30 | - | - | - |
| HM pectin | - | 30 | - | - | - | - | - | - | - | - | - | - | - | - |
| Dextran 40 | - | - | 60 | - | - | - | - | - | - | - | - | 60 | - | - |
| Dextran 70 | - | - | - | 60 | - | - | - | - | - | - | - | - | - | - |
| Starch | - | - | - | - | 60 | - | - | - | - | - | - | - | 60 | - |
| Pullulan | - | - | - | - | - | 90 | - | - | - | - | - | - | - | 90 |
| Glucose | - | - | - | - | - | - | 30 | - | - | - | - | - | - | - |
| Raffinose | - | - | - | - | - | - | - | - | - | - | - | - | - | - |
| Trehalose | - | - | - | - | - | - | - | - | - | - | - | - | - | - |
| Maltitol | - | - | - | - | - | - | - | - | - | - | - | - | - | - |
| Isomalt | - | - | - | - | - | - | - | - | - | - | - | - | - | - |
| Sorbitol | - | - | - | - | - | - | - | - | - | - | - | - | - | - |
| Maltodextrin | - | - | - | - | - | - | - | 30 | - | - | - | - | - | - |
| Dextran 70 | - | - | - | - | - | - | - | - | 30 | - | - | - | - | - |
| PVP K25 | - | - | - | - | - | - | - | - | - | 30 | - | - | - | - |
| Mannitol | - | - | - | - | - | - | - | - | - | - | 10 | 10 | 10 | 10 |
| Purified water | [969.9] | [969.9] | [939.9] | [939.9] | [939.9] | [909.9] | [969.9] | [969.9] | [969.9] | [969.9] | [959.9] | [929.9] | [929.9] | [899.9] |
| Aliquot [g/vial] | 1.0 | 1.0 | 1.0 | 1.0 | 1.0 | 1.0 | 1.0 | 1.0 | 1.0 | 1.0 | 1.0 | 1.0 | 1.0 | 1.0 |

(continued)

| Ingredients | Composition [parts by weight] | | | | | | | | | | | | | |
|---|---|---|---|---|---|---|---|---|---|---|---|---|---|---|
| | Comparative Examples | | | | | | | | | | | | | |
| | 1 | 2 | 3 | 4 | 5 | 6 | 7 | 8 | 9 | 10 | 11 | 12 | 13 | 14 |
| Composition | Group A | | | | | | Group B | | | | Groups A + [B'] | | | |

Table 9

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Example 1 | Groups A+B | 5 | 5 | 4 | 3 | 3 | 3 | 23 |
| Example 2 | | 5 | 5 | 4 | 3 | 3 | 3 | 23 |
| Example 3 | | 5 | 5 | 4 | 3 | 3 | 3 | 23 |
| Example 4 | | 5 | 5 | 4 | 3 | 3 | 3 | 23 |
| Example 5 | | 5 | 5 | 4 | 3 | 3 | 3 | 23 |
| Example 6 | | 5 | 4 | 4 | 3 | 3 | 3 | 22 |
| Example 7 | | 5 | 5 | 4 | 3 | 3 | 3 | 23 |
| Example 8 | | 5 | 5 | 4 | 3 | 3 | 3 | 23 |
| Example 9 | | 5 | 5 | 4 | 3 | 3 | 3 | 23 |
| Example 10 | | 5 | 5 | 4 | 3 | 3 | 3 | 23 |
| Example 11 | | 5 | 5 | 4 | 3 | 3 | 3 | 23 |
| Example 12 | | 5 | 5 | 4 | 3 | 3 | 3 | 23 |
| Example 13 | | 5 | 5 | 4 | 3 | 3 | 3 | 23 |
| Example 14 | | 5 | 5 | 4 | 3 | 3 | 3 | 23 |
| Example 15 | | 5 | 4 | 4 | 3 | 3 | 3 | 22 |
| Example 16 | | 5 | 4 | 4 | 3 | 3 | 3 | 22 |
| Example 17 | | 5 | 4 | 4 | 3 | 3 | 3 | 22 |
| Example 18 | | 5 | 4 | 4 | 3 | 3 | 3 | 22 |
| Comparative Example 1 | Group A | 5 | 4 | 3 | 3 | 1 | 3 | 19 |
| Comparative Example 2 | | 5 | 4 | 3 | 3 | 1 | 3 | 19 |
| Comparative Example 3 | | 4 | 4 | 3 | 3 | 1 | 3 | 78 |
| Comparative Example 4 | | 4 | 4 | 3 | 3 | 1 | 3 | 18 |
| Comparative Example 5 | | 4 | 3 | 2 | 3 | 1 | 3 | 16 |
| Comparative Example 6 | | 4 | 3 | 2 | 3 | 1 | 3 | 16 |
| Comparative Example 7 | Group B | 4 | 4 | 2 | 1 | 1 | 3 | 15 |
| Comparative Example 8 | | 4 | 3 | 2 | 1 | 1 | 3 | 14 |
| Comparative Example 9 | | 4 | 3 | 2 | 3 | 1 | 3 | 16 |
| Comparative Example 10 | | 4 | 4 | 3 | 3 | 1 | 3 | 18 |

(continued)

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Comparative Example 11 | Group A + [B'] | 4 | 3 | 2 | 3 | 3 | 3 | 18 |
| Comparative Example 12 | | 3 | 3 | 2 | 3 | 3 | 3 | 17 |
| Comparative Example 13 | | 3 | 2 | 2 | 3 | 3 | 3 | 16 |
| Comparative Example 14 | | 3 | 2 | 1 | 3 | 3 | 3 | 15 |

As shown in Table 9, the pharmaceutical compositions of Comparative Examples 1 to 10 consisting of only one additive from group (A) or group (B) showed poor stability of the allergen and deteriorated properties at day 90 of storage, which would cause problems in use. However, the pharmaceutical compositions of Examples showed improvement in the stability of the allergen and the stability of the properties by the combined use of additives from group (A) and group (B).

```
Group (B) + Group (C) (sugars, maltodextrin, PVP + viscous
polysaccharides)
```

(Example 19)

Raffinose (50 parts by weight) and guar gum (5 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.
Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2 °C for 90 days, and the allergenic activity and the properties after storage were evaluated by the same method as in Example 1. The solubility in water was also evaluated by the same method as in Example 1. Table 12 shows the results expressed as scores.

(Examples 20 to 28)

Allergen-containing preparation solutions were prepared with the compositions shown in Table 10 by the same procedure as in Example 19, and lyophilized to prepare pharmaceutical compositions. The pharmaceutical compositions obtained in Examples 20 to 28 were evaluated in the same manner as in Example 1, and the results were expressed as scores. Table 12 shows the results.

Table 10

| Ingredients | Composition [parts by weight] | | | | | | | | | |
|---|---|---|---|---|---|---|---|---|---|---|
| | Examples | | | | | | | | | |
| | 19 | 20 | 21 | 22 | 23 | 24 | 25 | 26 | 27 | 28 |
| Lyophilized dry powder of cedar pollen extract | 0.1 | 0.1 | 0.1 | 0.1 | 0.1 | 0.1 | 0.1 | 0.1 | 0.1 | 0.1 |
| Raffinose | 50 | 50 | 50 | 50 | 50 | 50 | - | - | - | - |
| Maltodextrin | - | - | - | - | - | - | 50 | 50 | 50 | - |
| PVP K25 | - | - | - | - | - | - | - | - | 50 | 50 |
| Guar gum | 5 | - | - | - | - | - | 5 | - | 5 | - |
| Locust bean gum | - | 5 | - | - | - | - | - | 5 | - | 5 |
| Xanthan gum | - | - | 5 | - | - | - | - | - | - | - |
| Tamarind gum | - | - | - | 5 | - | - | - | - | - | - |
| τ- Carrageenan | - | - | - | - | 5 | - | - | - | - | - |
| Deacylated gellan gum | - | - | - | - | - | 5 | - | - | - | - |
| Purfiied water | [944.9] | [944.9] | [944.9] | [944.9] | [944.9] | [944.9] | [944.9] | [944.9] | [944.9] | [944.9] |
| Aliquot [g/ vial] | 1.0 | 1.0 | 1.0 | 1.0 | 1.0 | 1.0 | 1.0 | 1.0 | 1.0 | 1.0 |
| Composition | Groups B + C | | | | | | | | | |

At Least One Additive from Group (C), or Group (B) + Group (C') (sugars, polysaccharides, PVP + additives that are viscous polysaccharides but have no stabilizing effect)

(Comparative Example 15)

Guar gum (10 parts by weight) was added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution. Subsequently, the obtained preparation solution was quickly dispensed in 1.0 g aliquots into a vial for lyophilization, and lyophilized to prepare a pharmaceutical composition. The obtained pharmaceutical composition was evaluated in the same manner as in Example 1, and the results were expressed as scores. Table 12 shows the results.

(Comparative Examples 16 to 20)

Solutions were prepared with the compositions shown in Table 11 by the same procedure as in Comparative Example 15, and lyophilized to prepare pharmaceutical compositions. The pharmaceutical compositions obtained in Comparative Examples 16 to 20 were evaluated in the same manner as in Example 1, and the results were expressed as scores. Table 12 shows the results.

(Comparative Example 21)

Raffinose (50 parts by weight) and κ-carrageenan (5 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.

Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2°C for 90 days, and the allergenic activity and the properties after storage were evaluated by the same method as in Example 1. The solubility in water was also evaluated by the same method as in Example 1. Table 12 shows the results expressed as scores.

(Comparative Examples 22 and 23)

Solutions were prepared with the compositions shown in Table 11 by the same procedure as in Comparative Example 21, and lyophilized to prepare pharmaceutical compositions. The pharmaceutical compositions obtained in Comparative Examples 22 and 23 were evaluated in the same manner as in Example 1, and the results were expressed as scores. Table 12 shows the results.

Table 11

| Ingredients | Composition [parts by weight] | | | | | | | | |
|---|---|---|---|---|---|---|---|---|---|
| | Comparative Examples | | | | | | | | |
| | 15 | 16 | 17 | 18 | 19 | 20 | 21 | 22 | 23 |
| Lyophilized dry powder of cedar pollen extract | 0.1 | 0.1 | 0.1 | 0.1 | 0.1 | 0.1 | 0.1 | 0.1 | 0.1 |
| Raffinose | - | - | - | - | - | - | 50 | - | - |
| Maltodextrin | - | - | - | - | - | - | - | 50 | - |
| PVP K25 | - | - | - | - | - | - | - | - | 50 |
| Guar gum | 10 | - | - | - | - | - | - | - | - |
| Locust bean gum | - | 10 | - | - | - | - | - | - | - |
| Xanthan gum | - | - | 10 | - | - | - | - | - | - |
| Tamarind gum | - | - | - | 10 | - | - | - | - | - |
| τ-Carrageenan | - | - | - | - | 10 | - | - | - | - |
| Deacylated gellan gum | - | - | - | - | - | 10 | - | - | - |
| κ-Carrageenan | - | - | - | - | - | - | 5 | 5 | 5 |
| Purified water | [989.9] | [989.9] | [989.9] | [989.9] | [989.9] | [989.9] | [944.9] | [944.9] | [944.9] |
| Aliquot [g/vial] | 1.0 | 1.0 | 1.0 | 1.0 | 1.0 | 1.0 | 1.0 | 1.0 | 1.0 |
| Composition | Group C | | | | | | Groups B + [C'] | | |

Table 12

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Example 19 | Groups B + C | 5 | 5 | 4 | 3 | 3 | 3 | 23 |
| Example 20 | | 5 | 5 | 4 | 3 | 3 | 3 | 23 |
| Example 21 | | 5 | 5 | 4 | 3 | 3 | 3 | 23 |
| Example 22 | | 5 | 5 | 4 | 3 | 3 | 2 | 22 |
| Example 23 | | 5 | 4 | 4 | 3 | 3 | 2 | 21 |
| Example 24 | | 5 | 4 | 4 | 3 | 3 | 2 | 21 |
| Example 25 | | 5 | 5 | 4 | 3 | 3 | 3 | 23 |
| Example 26 | | 5 | 5 | 4 | 3 | 3 | 3 | 23 |
| Example 27 | | 5 | 5 | 4 | 3 | 3 | 3 | 23 |
| Example 28 | | 5 | 5 | 4 | 3 | 3 | 3 | 23 |
| Comparative Example 15 | Group C | 5 | 4 | 3 | 1 | 1 | 1 | 15 |
| Comparative Example 16 | | 5 | 4 | 3 | 1 | 1 | 1 | 15 |
| Comparative Example 17 | | 4 | 4 | 3 | 1 | 1 | 1 | 14 |
| Comparative Example 18 | | 4 | 4 | 3 | 3 | 1 | 2 | 17 |
| Comparative Example 19 | | 4 | 3 | 2 | 1 | 1 | 1 | 12 |
| Comparative Example 20 | | 3 | 3 | 1 | 1 | 1 | 1 | 10 |
| Comparative Example 21 | Groups B + [C'] | 4 | 3 | 2 | 3 | 1 | 2 | 15 |
| Comparative Example 22 | | 3 | 2 | 1 | 3 | 1 | 2 | 12 |
| Comparative Example 23 | | 3 | 3 | 2 | 3 | 1 | 2 | 14 |

As shown in Table 12, the pharmaceutical compositions of Comparative Examples 15 to 20 consisting of only one additive from group (C) showed slightly higher stability of the allergen than the pharmaceutical compositions shown in Table 9, which consist of other additives. However, many of these pharmaceutical compositions have poor properties, problems in use, and an unsatisfactory solubility in water.
On the other hand, the pharmaceutical compositions of Examples showed that the use of an additive from group (B) having high solubility in water in combination with an additive from group (C) improved the stability of the allergen, the stability of properties, and the solubility in water, thus allowing easy sensitization to the antigen in the oral cavity.

```
Group (A) + Group (C) (polysaccharides having high formability
+ viscous polysaccharides)
```

(Example 29)

LM pectin (30 parts by weight) and guar gum (5 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.

Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2 °C for 90 days, and the allergenic activity and the properties after storage were evaluated by the same method as in Example 1. The solubility in water was also evaluated by the same method as in Example 1. Table 15 shows the results expressed as scores.

(Examples 30 to 42)

Solutions were prepared with the compositions shown in Table 13 by the same procedure as in Example 29, and lyophilized to prepare pharmaceutical compositions. The pharmaceutical compositions obtained in Examples 30 to 42 were evaluated in the same manner as in Example 1, and the results were expressed as scores. Table 15 shows the results.

```
Group (A) + Group (C') (polysaccharides having high formability
+ additives that are viscous polysaccharides but have no
stabilizing effect)
```

(Comparative Example 24)

LM pectin (30 parts by weight) and κ-carrageenan (5 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6.5. Further, purified water was added to the mixture to adjust the total weight to 1000 parts by weight, thereby obtaining an allergen-containing preparation solution.

Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2°C for 90 days, and the allergenic activity and the properties after storage were evaluated by the same method as in Example 1. The solubility in water was also evaluated by the same method as in Example 1. Table 15 shows the results expressed as scores.

(Comparative Examples 25 to 29)

Solutions were prepared with the compositions shown in Table 14 by the same procedure as in Comparative Example 24, and lyophilized to prepare pharmaceutical compositions. The pharmaceutical compositions obtained in Comparative Examples 25 to 29 were evaluated in the same manner as in Example 1, and the results were expressed as scores. Table 15 shows the results.

Table 13

| Ingredients | Composition [parts by weight] | | | | | | | | | | | | | |
|---|---|---|---|---|---|---|---|---|---|---|---|---|---|---|
| | Examples | | | | | | | | | | | | | |
| | 29 | 30 | 31 | 32 | 33 | 34 | 35 | 36 | 37 | 38 | 39 | 40 | 41 | 42 |
| Lyophilized dry powder of cedar pollen extract | 0.1 | 0.1 | 0.1 | 0.1 | 0.1 | 0.1 | 0.1 | 0.1 | 0.1 | 0.1 | 0.1 | 0.1 | 0.1 | 0.1 |
| LM pectin | 30 | 30 | 30 | 30 | - | - | - | - | - | - | - | - | - | - |
| HM pectin | - | - | - | - | 30 | 30 | - | - | - | - | - | - | - | - |
| Dextran 40 | - | - | - | - | - | - | 60 | 60 | - | - | - | - | - | - |
| Dextran 70 | - | - | - | - | - | - | - | - | 60 | 60 | - | - | - | - |
| Starch | - | - | - | - | - | - | - | - | - | - | 60 | 60 | - | - |
| Pullulan | - | - | - | - | - | - | - | - | - | - | - | - | 90 | 90 |
| Guar gum | 5 | - | - | - | 5 | - | 5 | - | 5 | - | 5 | - | 5 | - |
| Locust bean gum | - | 5 | - | - | - | 5 | - | 5 | - | 5 | - | - | - | - |
| Xanthan gum | - | - | 5 | - | - | - | - | - | - | - | - | - | - | - |
| Tamarind gum | - | - | - | 5 | - | - | - | - | - | - | - | 5 | - | 5 |
| $\kappa$-Carrageenan | - | - | - | - | - | - | - | - | - | - | - | - | - | - |
| Purified water | [964.9] | [964.9] | [964.9] | [964.9] | [964.9] | [964.9] | [964.9] | [964.9] | [964.9] | [964.9] | [934.9] | [934.9] | [904.9] | [904.9] |
| Aliquot [g/vial] | 1.0 | 1.0 | 1.0 | 1.0 | 1.0 | 1.0 | 1.0 | 1.0 | 1.0 | 1.0 | 1.0 | 1.0 | 1.0 | 1.0 |
| Composition | Groups A + C | | | | | | | | | | | | | |

Table 14

| Ingredients | Composition [parts by weight] | | | | | |
|---|---|---|---|---|---|---|
| | Comparative Examples | | | | | |
| | 24 | 25 | 26 | 27 | 28 | 29 |
| Lyophilized dry powder of cedar pollen extract | 0.1 | 0.1 | 0.1 | 0.1 | 0.1 | 0.1 |
| LM pectin | 30 | - | - | - | - | - |
| HM pectin | - | 30 | - | - | - | - |
| Dextran 40 | - | - | 60 | - | - | - |
| Dextran 70 | - | - | - | 60 | - | - |
| Starch | - | - | - | - | 60 | - |
| Pullulan | - | - | - | - | - | 90 |
| Guar gum | - | - | - | - | - | - |
| Locust bean gum | - | - | - | - | - | - |
| Xanthan gum | - | - | - | - | - | - |
| Tamarind gum | - | - | - | - | - | - |
| $\kappa$-Carrageenan | 5 | 5 | 5 | 5 | 5 | 5 |
| Purified water | [964.9] | [964.9] | [934.9] | [934.9] | [934.9] | [904.9] |
| Aliquot [g/vial] | 1.0 | 1.0 | 1.0 | 1.0 | 1.0 | 1.0 |
| Composition | Groups A + [C'] | | | | | |

Table 15

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Example 29 | Groups A + C | 5 | 5 | 5 | 3 | 3 | 3 | 24 |
| Example 30 | | 5 | 5 | 5 | 3 | 3 | 3 | 24 |
| Example 31 | | 5 | 5 | 4 | 3 | 3 | 3 | 23 |
| Example 32 | | 5 | 5 | 4 | 3 | 3 | 3 | 23 |
| Example 33 | | 5 | 5 | 5 | 3 | 3 | 3 | 24 |
| Example 34 | | 5 | 5 | 5 | 3 | 3 | 3 | 24 |
| Example 35 | | 5 | 5 | 4 | 3 | 3 | 3 | 23 |
| Example 36 | | 5 | 5 | 4 | 3 | 3 | 3 | 23 |
| Example 37 | | 5 | 5 | 4 | 3 | 3 | 3 | 23 |
| Example 38 | | 5 | 5 | 4 | 3 | 3 | 3 | 23 |
| Example 39 | | 5 | 5 | 4 | 3 | 3 | 3 | 23 |
| Example 40 | | 5 | 5 | 4 | 3 | 3 | 3 | 23 |
| Example 41 | | 5 | 5 | 4 | 3 | 3 | 3 | 23 |
| Example 42 | | 5 | 5 | 4 | 3 | 3 | 3 | 23 |

(continued)

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Comparative Example 24 | Groups A + [C'] | 4 | 3 | 2 | 3 | 1 | 2 | 15 |
| Comparative Example 25 | | 4 | 3 | 2 | 3 | 1 | 2 | 15 |
| Comparative Example 26 | | 3 | 3 | 2 | 3 | 1 | 2 | 14 |
| Comparative Example 27 | | 3 | 3 | 2 | 3 | 1 | 2 | 14 |
| Comparative Example 28 | | 3 | 2 | 1 | 3 | 1 | 2 | 12 |
| Comparative Example 29 | | 3 | 2 | 1 | 3 | 1 | 2 | 12 |

As shown in Table 15, the pharmaceutical compositions of Examples showed that the combined use of an additive from group (C) and an additive from group (A) improved the stability of the allergen, the stability of properties, and the solubility in water, thus allowing easy sensitization to the antigen in the oral cavity. In particular, the use of pectin and guar gum or locust bean gum (both are galactomannans) was found to show high stability of the allergen.

```
Group (A) + Group (B) + Group (C)
```

(Example 43)

LM pectin (30 parts by weight), raffinose (10 parts by weight), and guar gum (5 parts by weight) were added to purified water (850 parts by weight), and dissolved therein at a temperature of 40 to 80°C. After dissolution, the mixture was cooled to room temperature. Separately, cedar pollen extract lyophilized powder (10 parts by weight, manufactured by LSL) was added to purified water (30 parts by weight), and dissolved therein at room temperature. Subsequently, an allergen aqueous solution (4 parts by weight) was added to the above-obtained solution (in such a manner that the amount of the cedar pollen extract lyophilized powder in the solution would be 0.1 parts by weight) and quickly mixed, and it was made sure that there was no re-gelation. Using a pH adjuster (sodium hydroxide), the pH was adjusted to 6. 5. Further, purified water was added to the mixture to adjust the total weight to 1000 parts by weight, thereby obtaining an allergen-containing preparation solution.

Subsequently, the obtained preparation solution was quickly dispensed in 1.0 g aliquots into a vial for lyophilization, and lyophilized to prepare a pharmaceutical composition. The obtained pharmaceutical composition was stored at 40 ± 2 °C for 90 days, and the allergenic activity and the properties after storage were evaluated by the same method as in Example 1. The solubility in water was also evaluated by the same method as in Example 1. Table 17 shows the results expressed as scores.

(Examples 44 to 56)

Solutions were prepared with the compositions shown in Table 16 by the same procedure as in Example 44, and lyophilized to prepare pharmaceutical compositions. The pharmaceutical compositions obtained in Examples 44 to 56 were evaluated in the same manner as in Example 1, and the results were expressed as scores. Table 17 shows the results.

Table 16

| Ingredients | Composition [parts by weight] | | | | | | | | | | | | | |
| --- | --- | --- | --- | --- | --- | --- | --- | --- | --- | --- | --- | --- | --- | --- |
| | Examples | | | | | | | | | | | | | |
| | 43 | 44 | 45 | 46 | 47 | 48 | 49 | 50 | 51 | 52 | 53 | 54 | 55 | 56 |
| Lyophilized dry powder of cedar pollen extract | 0.1 | 0.1 | 0.1 | 0.1 | 0.1 | 0.1 | 0.1 | 0.1 | 0.1 | 0.1 | 0.1 | 0.1 | 0.1 | 0.1 |
| LM pectin | 30 | 30 | 30 | 30 | - | - | - | - | - | - | - | - | - | - |
| HM pectin | - | - | - | - | 30 | 30 | - | - | - | - | - | - | - | - |
| Dextran 40 | - | - | - | - | - | - | 60 | 60 | - | - | - | - | - | - |
| Dextran 70 | - | - | - | - | - | - | - | - | 60 | 60 | - | - | - | - |
| Starch | - | - | - | - | - | - | - | - | - | - | 60 | 60 | - | - |
| Pullulan | - | - | - | - | - | - | - | - | - | - | - | - | 90 | 90 |
| Raffinose | 10 | - | - | - | 10 | - | 10 | - | 10 | - | 10 | - | 10 | - |
| Maltodextrin | - | 10 | - | - | - | 10 | - | - | - | - | - | - | - | - |
| Dextran 70 | - | - | 10 | - | - | - | - | - | - | - | - | - | - | - |
| PVP K25 | - | - | - | 10 | - | - | - | 10 | - | 10 | - | 10 | - | 10 |
| Guar gum | 5 | 5 | - | - | 5 | - | 5 | - | 5 | - | 5 | - | 5 | - |
| Locust bean gum | - | - | 5 | 5 | - | 5 | - | 5 | - | 5 | - | 5 | - | 5 |
| Purified water | [954.9] | [954.9] | [954.9] | [954.9] | [954.9] | [954.9] | [924.9] | [924.9] | [924.9] | [924.9] | [924.9] | [924.9] | [894.9] | [894.9] |
| Aliquot [g/vial] | 1.0 | 1.0 | 1.0 | 1.0 | 1.0 | 1.0 | 1.0 | 1.0 | 1.0 | 1.0 | 1.0 | 1.0 | 1.0 | 1.0 |
| Composition | Groups A + B + C | | | | | | | | | | | | | |

EP 2 614 817 A1

Table 17

| Sample | | Remaining allergenic activity | | | Properties | | Solubility | Total |
|---|---|---|---|---|---|---|---|---|
| | | Day 14 | Day 30 | Day 90 | Day 0 | Day 90 | Water [37°C] | |
| Example 43 | Groups A + B + C | 5 | 5 | 5 | 3 | 3 | 3 | 24 |
| Example 44 | | 5 | 5 | 5 | 3 | 3 | 3 | 24 |
| Example 45 | | 5 | 5 | 5 | 3 | 3 | 3 | 24 |
| Example 46 | | 5 | 5 | 5 | 3 | 3 | 3 | 24 |
| Example 47 | | 5 | 5 | 5 | 3 | 3 | 3 | 24 |
| Example 48 | | 5 | 5 | 5 | 3 | 3 | 3 | 24 |
| Example 49 | | 5 | 5 | 5 | 3 | 3 | 3 | 24 |
| Example 50 | | 5 | 5 | 5 | 3 | 3 | 3 | 24 |
| Example 51 | | 5 | 5 | 5 | 3 | 3 | 3 | 24 |
| Example 52 | | 5 | 5 | 5 | 3 | 3 | 3 | 24 |
| Example 53 | | 5 | 5 | 5 | 3 | 3 | 3 | 24 |
| Example 54 | | 5 | 5 | 5 | 3 | 3 | 3 | 24 |
| Example 55 | | 5 | 5 | 5 | 3 | 3 | 3 | 24 |
| Example 56 | | 5 | 5 | 5 | 3 | 3 | 3 | 24 |

As shown in Table 17, the combined use of all additives from group (A), group (B), and group (C) improved the stability of the allergen, the stability of properties, and the solubility in water, and allowed easy sensitization to the antigen in the oral cavity.

The pharmaceutical composition of the present invention contains specific non-gelatin additives in combination with an allergen, and thus has excellent storage stability for preservation and delivery of the allergen.

Additionally, according to the method for producing the pharmaceutical composition of the present invention, even an allergen known to have very poor thermal stability can be stably maintained during production, and the resulting pharmaceutical composition also has excellent storage stability.

**Claims**

1. A pharmaceutical composition comprising:

   an allergen; and
   additives selected from at least two groups from among group (A) consisting of polysaccharides having high formability; group (B) consisting of mono- to hexasaccharides, sugar alcohols thereof, maltodextrin, and polyvinylpyrrolidone; and group (C) consisting of viscous polysaccharides;
   wherein the additives have a stabilizing effect on the allergen.

2. The pharmaceutical composition according to claim 1, free of water.

3. The pharmaceutical composition according to claim 1 or 2,
   wherein the additive selected from the polysaccharides having high formability in group (A) is at least one selected from the group consisting of pectin, dextran, starch, and pullulan.

4. The pharmaceutical composition according to claim 1, 2, or 3,
   wherein the additive selected from the mono- to hexasaccharides and sugar alcohols thereof in group (B) is at least one selected from the group consisting of glucose, mannose, raffinose, trehalose, maltitol, isomalt, and sorbitol.

5. The pharmaceutical composition according to claim 1, 2, 3, or 4,
wherein the additive selected from the viscous polysaccharides in group (C) is at least one selected from the group consisting of guar gum, tara gum, locust bean gum, xanthan gum, tamarind gum, τ-carrageenan, and gellan gum.

6. The pharmaceutical composition according to claim 1, 2, 3, 4, or 5,
wherein the allergen is a cedar pollen allergen protein.

7. The pharmaceutical composition according to claim 1, 2, 3, 4, 5, or 6, further comprising an organic acid salt.

8. A method for producing a pharmaceutical composition, the method comprising:

preparing an allergen-containing preparation solution in which an allergen and additives are dissolved in water; and
lyophilizing the allergen-containing preparation solution,
wherein the additives have a stabilizing effect on the allergen and are selected from at least two groups from among group (A) consisting of polysaccharides having high formability; group (B) consisting of mono- to hexasaccharides, sugar alcohols thereof, maltodextrin, and polyvinylpyrrolidone; and group (C) consisting of viscous polysaccharides.

9. The method for producing a pharmaceutical composition according to claim 8,
wherein the allergen-containing preparation solution has a pH in the range of 5.0 to 9.0.

**EUROPEAN SEARCH REPORT**

Europäisches
Patentamt
European
Patent Office
Office européen
des brevets

Application Number

EP 13 00 0090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 130 619 A2 (CHEMO SERO THERAPEUT RES INST [JP]) 9 January 1985 (1985-01-09)<br>* abstract; claims *<br>* page 5, lines 4-24 *<br>----- | 1-4,7-9 | INV.<br>A61K9/16<br>A61K9/20<br>A61K9/50 |
| X | WO 01/56611 A1 (NOVARTIS NUTRITION AG [CH]; SCHNEIDER HEINZ [CH])<br>9 August 2001 (2001-08-09)<br>* example 2 *<br>----- | 1-9 | |
| X | EP 0 192 321 A2 (BEECHAM GROUP PLC [GB]) 27 August 1986 (1986-08-27)<br>* example 2 *<br>----- | 1-9 | |
| X | EP 0 603 992 A1 (UNIV CINCINNATI [US]) 29 June 1994 (1994-06-29)<br>* page 3, lines 13-36, 42-44 *<br>----- | 1-9 | |

TECHNICAL FIELDS
SEARCHED      (IPC)

A61K

The present search report has been drawn up for all claims

| Place of search | Date of completion of the search | Examiner |
|---|---|---|
| Munich | 9 April 2013 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**
EP 13 00 0090

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2013

| Patent document cited in search report | | Publication date | Patent family member(s) | | Publication date |
|---|---|---|---|---|---|
| EP 0130619 | A2 | 09-01-1985 | AU | 572162 B2 | 05-05-1988 |
| | | | AU | 2999384 A | 10-01-1985 |
| | | | CA | 1237076 A1 | 24-05-1988 |
| | | | DE | 3477139 D1 | 20-04-1989 |
| | | | EP | 0130619 A2 | 09-01-1985 |
| | | | JP | H0437806 B2 | 22-06-1992 |
| | | | JP | S6013718 A | 24-01-1985 |
| WO 0156611 | A1 | 09-08-2001 | AR | 027523 A1 | 02-04-2003 |
| | | | AU | 3544201 A | 14-08-2001 |
| | | | JP | 2003521524 A | 15-07-2003 |
| | | | US | 2003003133 A1 | 02-01-2003 |
| | | | WO | 0156611 A1 | 09-08-2001 |
| EP 0192321 | A2 | 27-08-1986 | AU | 5245586 A | 24-07-1986 |
| | | | DK | 24986 A | 20-07-1986 |
| | | | EP | 0192321 A2 | 27-08-1986 |
| | | | GR | 860123 A1 | 19-05-1986 |
| | | | NZ | 214853 A | 12-02-1988 |
| | | | PT | 81867 A | 01-02-1986 |
| EP 0603992 | A1 | 29-06-1994 | AT | 143803 T | 15-10-1996 |
| | | | AU | 664222 B2 | 09-11-1995 |
| | | | CA | 2086631 A1 | 23-06-1994 |
| | | | DE | 69305313 D1 | 14-11-1996 |
| | | | DE | 69305313 T3 | 21-06-2001 |
| | | | DK | 0603992 T3 | 24-03-1997 |
| | | | EP | 0603992 A1 | 29-06-1994 |
| | | | ES | 2095001 T3 | 01-02-1997 |
| | | | GR | 3022090 T3 | 31-03-1997 |
| | | | GR | 3035452 T3 | 31-05-2001 |
| | | | JP | H0710771 A | 13-01-1995 |
| | | | NZ | 245618 A | 26-10-1994 |
| | | | SG | 52402 A1 | 28-09-1998 |
| | | | US | 5591433 A | 07-01-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006513269 T
- JP 3932272 B
- JP 2007277094 A